# EUROPEAN PATENT APPLICATION

(11) **EP 4 309 776 A1**
(43) Date of publication of application: **24.01.2024**
(21) Application number: 21907181.8
(22) Date of filing: 17.12.2021
(51) Int. Cl.: B01J 4/00, B01J 19/00, B01J 19/18, B01F 27/113, B01F 27/91, C08G 18/76

(54) **BATCH REACTION APPARATUS**

(30) Priority: 18.12.2020 KR 20200178880; 18.12.2020 KR 20200178881; 18.12.2020 KR 20200178882; 18.12.2020 KR 20200178883
(71) Applicant: Hanwha Solutions Corporation, Jung-gu Seoul 04541 (KR)
(72) Inventor: LEE, Hye Won, Daejeon 34128 (KR); RYU, Hyun Cheol, Daejeon 34128 (KR); HAN, Keedo, Daejeon 34128 (KR)
(74) Representative: Berggren Oy
(86) International application number: PCT/KR2021/019346
(87) International publication number: WO 2022/131877

(57) **Abstract**

A batch reactor according to an embodiment of the present disclosure includes: a reactor receiving a solvent in a liquid phase; a sparger configured to supply a raw material in a gas phase into the reactor; and a stirrer having an impeller on a rotational shaft installed in the reactor in a height direction to stir the solvent and the raw material, in which the impeller includes a radial-type impeller and an axial-type impeller.

## Description

### [Technical Field]

### CROSS-REFERENCE TO RELATED APPLICATION(S)

This application claims priority to and the benefit of Korean Patent Applications Nos. 10-2020-0178880, 10-2020-0178881, 10-2020-0178882, 10-2020-0178883 filed in the Korean Intellectual Property Office on December 18, 2020, and all of the disclosures in those Korean patent applications are hereby incorporated by reference as part of this specification.

The present invention relates to a batch reactor.

### [Background Art]

During a manufacturing process of xylylene diisocyanate, a batch reactor may be used to inject gaseous raw materials into a salt formation reaction solvent and mix them with an impeller.

In this case, the salt formation reaction is carried out in the presence of a solvent. Amine salts, which are products of the salt formation reaction, are in the form of particles with a diameter of several to tens of micrometers. As the reaction proceeds, the amine salts are dispersed in the solvent and turn into the form of a slurry.

In general, in heterogeneous reactions with these different phases, mixing of the reactants is very important. For example, in order to effectively disperse hydrogen chloride in a solvent in a liquid phase and dissolve hydrogen chloride rapidly, a method of blowing the gas into the liquid phase in the form of small bubbles has been tried to increase a contact area between the gas and the liquid.

In another example, the batch reactor may be used to inject phosgene in a gas phase into amine salts in a slurry phase and mix them with an impeller for the second phosgenation reaction among total two steps of the manufacturing process of xylylene diisocyanate.

In this case, the phosgene sprayed into the reactor need to be effectively distributed to the amine salts in the slurry phase to ensure a gas holdup within a certain time.

### [Disclosure]

### [Technical Problem]

One aspect of the present disclosure is to provide a batch reactor that improves reactivity of aliphatic isocyanate when the aliphatic isocyanate is reacted with gaseous hydrogen chloride.

In addition, there is provided a batch reactor that improves mixing performance of reactants during the reaction of aliphatic isocyanate with hydrogen chloride in a gas phase.

In addition, there is provided a batch reactor that improves distribution performance of hydrogen chloride to a solvent during the reaction of aliphatic isocyanate with hydrogen chloride in a gas phase.

In addition, there is provided a batch reactor that improves phosgenation reactivity of aliphatic isocyanate when the aliphatic isocyanate is reacted with a gas phase phosgene.

In addition, there is provided a batch reactor that improves mixing performance of phosgene with amine salt in a slurry phase during the reaction of aliphatic isocyanate using phosgene in a gas phase.

In addition, there is provided a batch reactor that improves distribution performance of phosgene to amine salt in a slurry phase during the reaction of aliphatic isocyanate with phosgene in a gas phase.

### [Technical Solution]

A batch reactor according to an embodiment of the present disclosure includes: a reactor receiving a solvent in a liquid phase; a sparger configured to supply a raw material in a gas phase into the reactor; and a stirrer having an impeller on a rotational shaft installed in the reactor in a height direction to stir the solvent and the raw material, in which the impeller includes a radial-type impeller and an axial-type impeller.

The radial-type impeller may be provided in one stage at a lower end of the rotational shaft, and the axial-type impeller may be provided in one or more stages on the rotational shaft at an upper side of the radial-type impeller.

The axial-type impeller may be provided in one stage at a lower end of the rotational shaft, and the radial-type impeller may be provided one or more stages on the rotational axis at an upper side of the axial-type impeller.

The reactor may include a baffle disposed in parallel with the rotational axis and installed on an inner surface of the reactor. The baffle may have a plate shape or a rod shape.

The raw material may be hydrogen chloride.

A batch reactor according to an embodiment of the present disclosure may supply hydrogen chloride, which is the raw material to produce xylylene diisocyanate.

Meanwhile, a batch reactor according to an embodiment of the present disclosure includes: a reactor receiving a solvent in a liquid phase; a stirrer having an impeller on a rotational shaft installed in the reactor in a height direction and rotating; and a sparger allowing a raw material to be distributed to the solvent in the reactor by an operation of the stirrer so that hydrogen chloride, which is the raw material in a gas phase, is supplied to the solvent to produce xylene diisocyanate, in which the sparger is configured to supply the raw material in the gas phase in at least one direction of an axial direction of the stirrer and a circumferential direction of the reactor.

The sparger may be provided in plural along a circumferential direction of the stirrer and installed on a sidewall portion of the reactor at a predetermined interval.

The sparger may include: a pipe installed in the reactor in a direction in which a length direction of the sparger intersects an axial direction of the stirrer; and a bubble formation portion provided at an end of the pipe to supply the raw material in the gas phase in a bubble state toward the impeller.

The sparger may be provided as one sparger at a lower end of the reactor and installed on a sidewall portion of the reactor.

The sparger may include: a pipe installed in the reactor with a length direction thereof being oriented in a direction of a diameter of the reactor; and a bubble formation portion provided at an end of the pipe to supply the raw material in the gas phase that is in a bubble state toward a downstream side of the impeller.

The sparger may include: a ring provided in a circular shape on the lower side of the stirrer; and a bubble formation portion provided on an upper side of the ring and configured to supply the raw material in the gas phase in a bubble state toward a downstream side of the impeller.

The sparger may be formed as a sintered porous type and installed in the reactor in a direction in which a length direction thereof intersects an axial direction of the stirrer to supply the raw material in the gas phase towards the impeller in a bubbled state.

The impeller may include: a radial-type impeller or an axial-type impeller installed at a lower end; and one or a plurality of impellers of the same or a different type installed on a top of the impeller installed on the lower end.

Meanwhile, a batch reactor according to an embodiment of the present disclosure includes: a reactor receiving amine salts in a slurry phase; a sparger configured to supply phosgene, which is a raw material in a gas phase, to the reactor; and a stirrer having an impeller on a rotational shaft installed in the reactor in a height direction to stir the amine salts and the phosgene to produce xylylene diisocyanate, in which the impeller is disposed in a plurality of stages on the rotational shaft.

The impeller may be formed as at least one of a radial-type impeller and an axial-type impeller.

The impeller may include a radial-type impeller provided in one stage at a lower end of the rotational shaft; and an axial-type impeller provided in at least two stages on the rotational shaft on an upper side of the radial-type impeller.

The impeller may include a radial-type impeller provided in one stage at a lower end of the rotational shaft; and an axial-type impeller provided in one stage on the rotational shaft on an upper side of the radial-type impeller.

The sparger may be installed to supply the raw material in the gas phase in an axial direction of the stirrer and in a circumferential direction of the reactor.

The sparger may include: a pipe installed on a sidewall portion of the reactor in a direction in which a length direction of the sparger intersects an axial direction of the stirrer; and a bubble formation portion provided with a plurality of spray ports at an end of the pipe to supply the raw material in the gas phase in a bubble state toward the impeller.

Meanwhile, a batch reactor according to an embodiment of the present disclosure includes: a reactor receiving amine salts in a slurry phase; a stirrer having an impeller on a rotational shaft installed in the reactor in a height direction and rotating; and a sparger configured to supply a raw material in a gas phase to the amine salts in the reactor to allow the raw material to be distributed to the amine salts by an operation of the stirrer, in which the sparger is provided in plural along one direction of an axial direction of the stirrer and a circumferential direction of the reactor to supply the raw material in the gas phase.

The sparger may be installed on a sidewall portion of the reactor at a predetermined interval along the axial direction of the stirrer.

The sparger may include: a pipe installed in the reactor in a direction in which a length direction of the sparger intersects an axial direction of the stirrer; and a bubble formation portion provided at an end of the pipe to supply the raw material in the gas phase in a bubble state toward the impeller.

The sparger may include: a guide path penetratively formed within the rotational shaft; and a bubble formation portion penetrating the rotational shaft and connected to the guide path to supply the raw material in the gas phase that is in a bubble state.

The impeller may be provided in a plurality of stages on the rotational shaft, and the bubble formation portion may be provided on a lower side of each of the impellers.

The sparger may be installed on a sidewall portion of the reactor at a predetermined interval along the circumferential direction of the reactor at a lower end of the reactor.

The sparger may include: a pipe installed in the reactor with a length direction thereof being oriented in a direction of a diameter of the reactor; and a bubble formation portion provided at an end of the pipe to supply the raw material in the gas phase in a bubble state toward a downstream side of the impeller.

A batch reactor according to an embodiment of the present disclosure may supply phosgene, which is the raw material, to produce xylylene diisocyanate.

The sparger may be formed as a sintered porous type and installed in the reactor in a direction in which a length direction thereof intersects an axial direction of the stirrer to supply the raw material in the gas phase towards the impeller in a bubbled state.

### [Advantageous Effect]

The batch reactor of one embodiment has a radial-type impeller and an axial-type impeller on the rotational shaft of the stirrer, so that the mixing performance of the solvent and hydrogen chloride, i.e., the gas holdup, can be improved during the reaction of aliphatic isocyanate with hydrogen chloride in the gas phase. Therefore, reactivity of aliphatic isocyanate using hydrogen chloride can be improved.

In addition, the batch reactor of one embodiment can improve distribution performance of hydrogen chloride to the solvent, that is, the gas holdup, in the reaction of aliphatic isocyanate with hydrogen chloride in the gas phase by supplying the raw material in the gas phase to the sparger in the axial direction of the stirrer or in the circumferential direction of the reactor, so that the raw material is distributed to the solvent by the operation of the stirrer. Therefore, reactivity of aliphatic isocyanate using hydrogen chloride can be improved.

In addition, since the batch reactor of one embodiment may be provided with the impeller in plural on the rotational axis of the stirrer, it is possible to improve mixing performance of phosgene to the amine salts in the slurry phase, that is, the gas holdup when the reaction of aliphatic isocyanate with phosgene in the gas phase. Therefore, aliphatic isocyanate with improved phosgenation reactivity can be prepared.

In addition, the batch reactor of an embodiment is provided with a plurality of spargers along the axial direction of the stirrer or the circumferential direction of the reactor to supply the raw material in the gas phase, which can improve distribution performance of the raw material to the amine salts in the slurry phase, that is, the gas holdup.

The batch reactor of an embodiment is provided with a plurality of spargers along the axial direction of the stirrer or the circumferential direction of the reactor and supplies phosgene in the gas phase to the impeller side, which can improve distribution performance, that is, the gas holdup, of phosgene to the amine salts in the slurry phase. Therefore, reactivity of aliphatic isocyanate using phosgene can be improved.

### [Description of the Drawings]

FIG. 1 is a cross-sectional view of a batch reactor according to Embodiment 1-1 of the present disclosure.
FIG. 2 is a cross-sectional view of a batch reactor according to Embodiment 1-2 of the present disclosure.
FIG. 3 is a cross-sectional view of a batch reactor according to Comparative Example 1-1.
FIG. 4 is a cross-sectional view of a batch reactor according to Comparative Example 1-2.
FIG. 5 is a cross-sectional view of a batch reactor according to Embodiment 1-3 of the present disclosure.
FIG. 6 is a cross-sectional view of a batch reactor according to Embodiment 1-4 of the present disclosure.
FIG. 7 is a cross-sectional view of a batch reactor according to Embodiment 1-5 of the present disclosure.
FIG. 8 is a cross-sectional view of a batch reactor according to Embodiment 2-1 of the present disclosure.
FIG. 9 is a cross-sectional view of a batch reactor according to Embodiment 2-2 of the present disclosure.
FIG. 10 is a cross-sectional view of a batch reactor according to Embodiment 2-3 of the present disclosure.
FIG. 11 is a cross-sectional view of a batch reactor according to Comparative Example 2.
FIG. 12 is a cross-sectional view of a batch reactor according to Embodiments 2-4 of the present disclosure.
FIG. 13 is a cross-sectional view of a batch reactor according to Embodiment 3-1 of the present disclosure.
FIG. 14 is a cross-sectional view of a batch reactor according to Embodiment 3-2 of the present disclosure.
FIG. 15 is a cross-sectional view of a batch reactor according to Comparative Example 3.
FIG. 16 is a cross-sectional view of a batch reactor according to Embodiment 4-1 of the present disclosure.
FIG. 17 is a cross-sectional view of a batch reactor according to Embodiment 4-2 of the present disclosure.
FIG. 18 is a cross-sectional view of a batch reactor according to Embodiment 4-3 of the present disclosure.
FIG. 19 is a cross-sectional view of a batch reactor according to Comparative Example 4.
FIG. 20 is a cross-sectional view of a batch reactor according to Embodiments 4-4 of the present disclosure.

### [Mode for Invention]

Hereinafter, embodiments of the present invention will be described in detail with reference to the accompanying drawings so that those with ordinary skill in the art to which the present invention pertains may easily carry out the embodiments. As those skilled in the art would realize, the described embodiments may be modified in various different ways, all without departing from the scope of the present invention. The drawings and description are to be regarded as illustrative in nature and not restrictive. Like reference numerals designate like elements throughout the specification.

Hereinafter, a first embodiment of the present disclosure will be described.

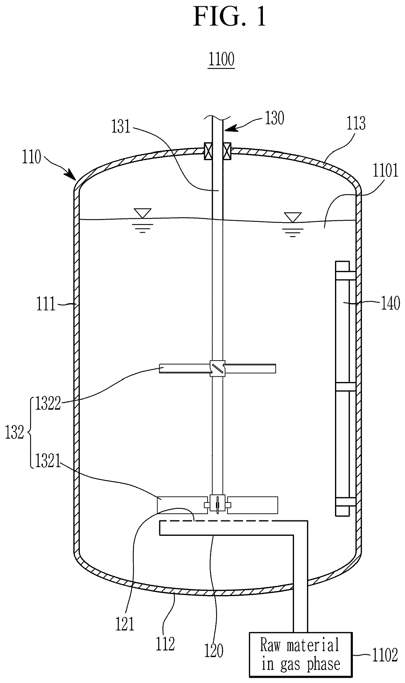

FIG. 1 is a cross-sectional view of a batch reactor according to Embodiment 1-1 of the present disclosure. With reference to FIG. 1, a batch reactor 1100 of Embodiment 1-1 includes a reactor 110 that receives a solvent 1101 in a liquid phase, a sparger 120 that supplies a raw material 1102 in a gas phase into the reactor 110, and a stirrer 130.

For an example, the reactor 110 includes a cylindrical sidewall portion 111 and a bottom portion 112 and a cover portion 113 that form lower and upper sides of the sidewall portion 111, respectively, to form a reaction space. The bottom portion 112 and the cover portion 113 are formed with convex curved surfaces on the lower and upper sides, so that the solvent 1101 in a liquid phase and the raw material 1102 in a gas phase may flow and be effectively mixed.

The sparger 120 extends upward from the bottom portion 112 of the reactor 110 and is installed at a lower portion of the stirrer 130 and has a plurality of spray ports 121 that are capable of spaying and supplying the raw material in the gas phase from the lower portion of the stirrer 130.

The sparger 120 is bent at the lower portion of the stirrer 130 and has the plurality of spray ports 121 in response to a range of rotation of the stirrer 130. In the sparger 120, the plurality of spray ports 121 enable uniform spraying of hydrogen chloride (HCl), the raw material 1102 in the gas phase, into the solvent 1101 in the liquid phase.

The stirrer 130 includes a rotational shaft 131 installed in the reactor 110 in a height direction, and an impeller 132 provided on the rotational shaft 131 to stir the solvent 1101 and the raw material 1102. The rotational shaft 131 is rotatably installed on the cover portion 113 of the reactor 110, a lower end thereof faces the bottom portion 112, and may be driven by an externally provided drive motor (not illustrated).

The impeller 132 includes a radial-type impeller 1321 and an axial-type impeller 1322. The radial-type impeller 1321 generates flow in a transverse direction perpendicular to the rotational shaft 131 to crush and disperse the raw material 1102 in the gas phase supplied in the solvent 1101 in the liquid phase. The axial-type impeller 1322 generates flow in the same axial direction as the rotational shaft 131 to crush and disperse the raw material 1102 in the gas phase supplied in the solvent 1101 in the liquid phase.

In Embodiment 1-1, the radial-type impeller 1321 is provided in one stage on a lower end of the rotational shaft 131, and the axial-type impeller 1322 may be provided in one or more stages on the rotational shaft 131 on an upper side of the radial-type impeller 1321. That is, the radial-type impeller 1321 is installed on a first stage, and the axial-type impeller 1322 is installed on a second stage. Although not illustrated more, the axial-type impeller 1322 may be installed in one or more stages depending on heights of the rotational shaft 131 and the reactor 110.

The radial-type impeller 1321 is rotationally driven on an upper side of the raw material 1102 in the gas phase being sprayed from the spray ports 121 of the sparger 120 to flow the solvent 1101 in the transverse direction. Therefore, the raw material 1102 in the gas phase being sprayed into the spray port 121 of the sparger 120 is mixed into the solvent 1101 while flowing in the transverse direction by the transverse flow of the solvent 1101.

The axial-type impeller 1322 is driven on an upper side of the radial-type impeller 1321 to flow the solvent 1101 in the axial direction. Therefore, the partially mixed raw material 1102 in the gas phase and the raw material 1102 in the gas phase are further mixed in the solvent 1101 while flowing in the axial direction by the axial flow of the solvent 1101.

As the salt formation reaction proceeds by mixing the raw material 1102 in the solvent 1101, amine salts are produced. The amine salts, which are products, are in the form of particles with a diameter of several to tens of micrometers, so as the reaction proceeds, the amine salts change into the form of a slurry that is dispersed in the solvent.

As described above, the mixed use of the radial-type impeller 1321 and the axial-type impeller 1322 can increase the gas holdup when the raw material 1102 in the gas phase is sprayed and mixed in the solvent 1101. Therefore, a reaction rate within the reactor 110 can be increased. In conclusion, in a reaction in which the raw material 1102 is mixed with the solvent 1101 to produce a slurry, a homogeneous product can be produced in a shorter time, thereby increasing productivity and improving stability of the product quality.

Meanwhile, the reactor 110 further includes a baffle 140 disposed in parallel with the rotational shaft 131 and installed on an inner surface, that is, an inner side of the sidewall portion 111. For example, the baffle 140 may be formed in the form of a plate having a predetermined width and height, or may be formed in the form of a rod having a predetermined diameter and length.

The baffle 140 creates a vortex in the flowing solvent 1101 when the impeller 132 is driven, allowing for more uniform mixing of the solvent 1101 and the raw material 1102 over the entire area of the reactor 110 in the height direction.

The baffles 140 are provided in plural along a circumferential direction inside the reactor 110 to repeatedly form vortices in the circumferential direction to allow for more uniform mixing of the solvent 1101 and raw material 1102 over the entire area in the circumferential direction.

In addition, the baffle 140 may be provided in a vortex area generated by the rotational drive of the radial-type impeller 1321 and the axial-type impeller 1322, which may directly act on the mixing of the solvent 1101 and the raw material 1102.

The use of the radial-type impeller 1321 and the axial-type impeller 1322 and the baffle 140 may further increase the gas holdup when the raw material 1102 in the gas phase is sprayed and mixed in solvent 1101. Therefore, the reaction rate within the reactor 110 may be further increased.

The batch reactor 1100 according to Embodiment 1-1 of the present disclosure is used for a reaction between a gas phase and a liquid phase, and can be used in a process to produce xylylene diisocyanate by supplying hydrogen chloride, which is the raw material 1102 in the gas phase, to a solvent 1101 in the liquid phase.

Hereinafter, various variation examples and comparative examples of the present embodiment will be described. The descriptions of configurations that are identical to Embodiment 1-1 are omitted, and the descriptions of configurations that are different are described.

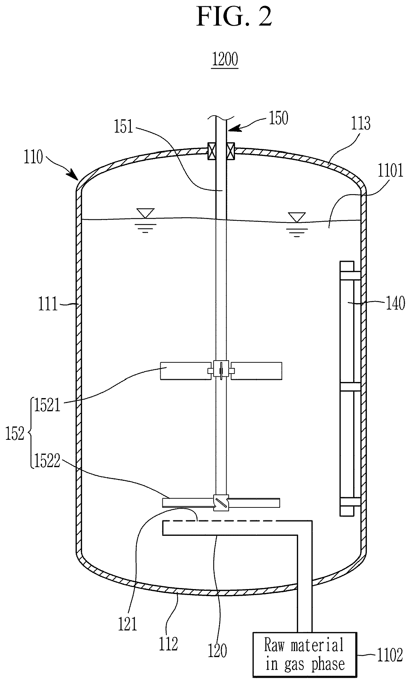

FIG. 2 is a cross-sectional view of a batch reactor according to Embodiment 1-2 of the present disclosure. With reference to FIG. 2, in an impeller 152 of a batch reactor 1200 of Embodiment 1-2, an axial-type impeller 1522 is provided in one stage on a lower end of a rotational shaft 151, and a radial-type impeller 1521 may be provided in one stage or two or more stages on the rotational shaft 151 on an upper side of the axial-type impeller 1522. That is, the axial-type impeller 1522 is installed at a first stage, and the radial-type impeller 1521 is installed at a second stage. Although not illustrated more, the radial-type impeller 1521 may be installed in one or more stages depending on heights of the rotational shaft 151 and the reactor 110.

The axial-type impeller 1522 is rotationally driven on the upper side of the raw material 1102 in the gas phase being sprayed from the spray ports 121 of the sparger 120 to flow the solvent 1101 in an axial direction. Therefore, the raw material 1102 in the gas phase being sprayed into the spray ports 121 of the sparger 120 is mixed into the solvent 1101 while flowing in the axial direction by the axial flow of the solvent 1101.

The radial-type impeller 1521 is driven on an upper side of the axial-type impeller 1522 to flow the solvent 1101 in the transverse direction. Therefore, the partially mixed raw material 1102 in the gas phase and the raw material 1102 in the gas phase are further mixed in the solvent 1101 while flowing in the transverse direction by the transverse flow of the solvent 1101.

As described above, the mixed use of the axial-type impeller 1522 and the radial-type impeller 1521 increases the gas holdup when the raw material 1102 in the gas phase is sprayed and mixed in the solvent 1101. Therefore, a reaction rate within the reactor 110 can be increased. In conclusion, in a reaction in which the raw material 1102 is mixed with the solvent 1101 to produce a slurry, a homogeneous product can be produced in a shorter time, thereby increasing productivity and improving stability of the product quality.

Further, the baffle 140 may be provided in a vortex area generated by the rotational drive of the axial-type impeller 1522 and the radial-type impeller 1521, which may directly act on the mixing of the solvent 1101 and the raw material 1102.

The use of the axial-type impeller 1522 and the radial-type impeller 1521 and the baffle 140 may further increase the gas holdup when the raw material 1102 in the gas phase is sprayed and mixed in the solvent 1101. Therefore, the reaction rate within the reactor 110 may be further increased.

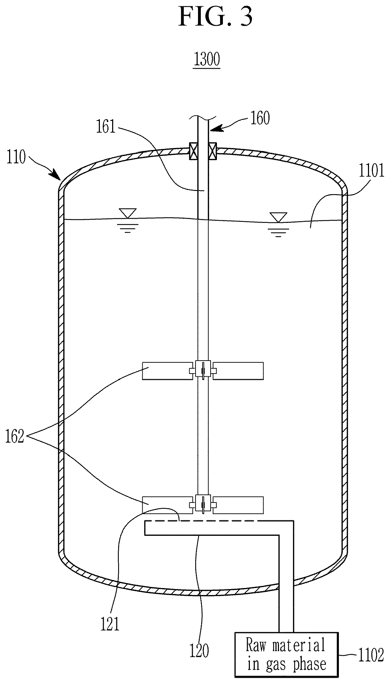

FIG. 3 is a cross-sectional view of a batch reactor according to Comparative Example 1-1. With reference to FIG. 3, in a batch reactor 1300 of Comparative Example 1-1, a stirrer 160 has a plurality of radial-type impellers 162 mounted on a rotational shaft 161.

The radial-type impeller 162 is rotationally driven on the upper side of the raw material 1102 in the gas phase sprayed from the sparger 120 to flow the solvent 1101 in the transverse direction. Therefore, the raw material 1102 in the gas phase being sprayed into the spray port 121 of the sparger 120 is mixed into the solvent 1101 while flowing in the transverse direction by the transverse flow of the solvent 1101.

The two or more radial-type impellers 162 are driven identically to mix the raw material 1102 in the gas phase in the solvent 1101 with the transverse flow of the solvent 1101. Compared to Embodiments 1-1 and 1-2, it is difficult to form a flow in the axial direction with respect to the solvent 1101 in Comparative Example 1-1.

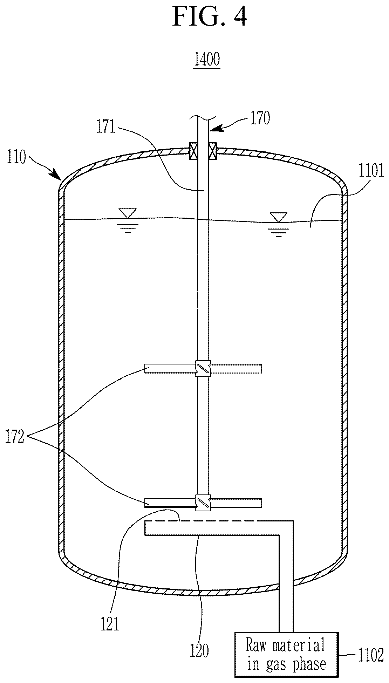

FIG. 4 is a cross-sectional view of a batch reactor according to Comparative Example 1-2. With reference to FIG. 4, in a batch reactor 1400 of Comparative Example 1-2, a stirrer 170 has a plurality of axial-type impellers 172 mounted on a rotational shaft 171.

The axial-type impeller 172 is rotationally driven on the upper side of the raw material 1102 in the gas phase sprayed from the sparger 120 to flow the solvent 1101 in the axial direction. Therefore, the raw material 1102 in the gas phase being sprayed into the spray ports 121 of the sparger 120 is mixed into the solvent 1101 while flowing in the axial direction by the axial flow of the solvent 1101.

The two or more axial-type impellers 172 are driven identically to mix the raw material 1102 in the gas phase in the solvent 1101 with the axial flow of the solvent 1101. Compared to Embodiments 1-1 and 1-2, it is difficult to form a flow in the transverse direction with respect to the solvent 1101 in Comparative Example 1-2.

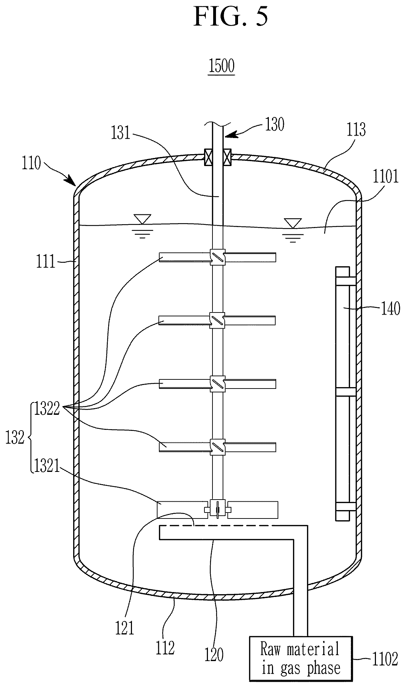

FIG. 5 is a cross-sectional view of a batch reactor according to Embodiment 1-3 of the present disclosure. With reference to FIG. 5, in the impeller 132 of a batch reactor 1500 of the Embodiment 1-3, the radial-type impeller 1321 is provided in one stage on the lower end of the rotational shaft 131, and the axial-type impeller 1322 is provided in four stages on the rotational shaft 131 from the upper side of the axial-type impeller 1322.

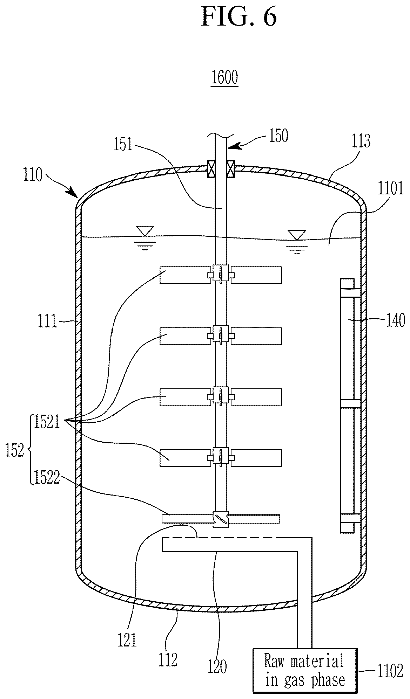

FIG. 6 is a cross-sectional view of a batch reactor according to Embodiment 1-4 of the present disclosure. With reference to FIG. 6, in the impeller 152 of a batch reactor 1600 of Embodiment 1-4, the axial-type impeller 1522 is provided in one stage on the lower end of the rotational shaft 151, and the radial-type impeller 1521 is provided in four stages on the rotational shaft 151 from the upper side of the axial-type impeller 1522.

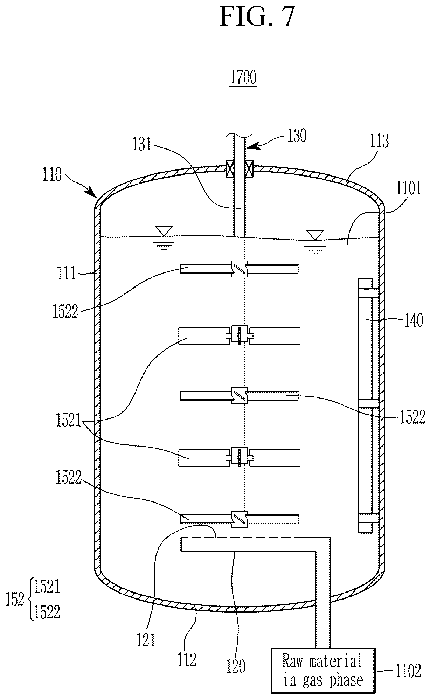

FIG. 7 is a cross-sectional view of a batch reactor according to Embodiment 1-5 of the present disclosure. With reference to FIG. 7, in the impeller 152 of the batch reactor 1500 of Embodiment 1-5, the axial-type impeller 1522 is provided in three stages at the lower, middle, and upper portions of the rotational shaft 151, and the radial-type impeller 1521 is provided in two stages between the stages of the axial-type impeller 1522.

Although not illustrated, the radial-type impeller may be provided in three stages at the lower, middle, and upper portions of the rotational shaft, and the axial-type impeller may be provided in two stages between the stages of the radial-type impeller.

Consequently, in the stirrer of the present disclosure, the impeller may be installed with a radial-type impeller or an axial-type impeller at the lower end and one, two or more, or a plurality of impellers of the same or different types may be provided at the top of the impeller installed at the lower end. In addition, in the stirrer, the impellers may be provided alternately up to the n^{th} stage, which requires a radial-type impeller and an axial-type impeller. The various drawings corresponding to these cases are omitted.

Table 1 shows the gas holdup for Embodiment 1-1 to Embodiment 1-5 and Comparative Example 1-1 and Comparative Example 1-2.

**(Table 1)**

| | Embodiment 1-1 | Embodiment 1-2 | Embodiment 1-3 | Embodiment 1-4 | Embodiment 1-5 | Comparative Example 1-1 | Comparative Example 1-2 |
|---|---|---|---|---|---|---|---|
| First stage impeller | Radial-type | Axial-type | Radial-type | Axial-type | Axial-type | Radial-type | Axial-type |
| Second stage impeller | Axial-type | Radial-type | Axial-type | Radial-type | Radial-type | Radial-type | Axial-type |
| Third stage impeller | - | - | Axial-type | Radial-type | Axial-type | - | - |
| Fourth stage impeller | - | - | Axial-type | Radial-type | Radial-type | - | - |
| Fourth stage impeller | - | - | Axial-type | Radial-type | Axial-type | - | - |
| Gas holdup (%) | >10 | >10 | >10 | >10 | >10 | 4.7 | 8.0 |
| Reaction time (hr) | 6.0 | 6.3 | 4.5 | 5.3 | 5.2 | 7.8 | 6.7 |

### Embodiment 1-1

The solvent 1101 was contained in the batch reactor 1100 of FIG. 1 , and hydrogen chloride, which is the raw material 1102, was supplied to the solvent 1101 by the sparger 120 to produce xylylene diisocyanate. In the Embodiment 1-1, one stage of radial-type impeller 1321 and one stage of axial-type impeller 1322 were used. The stirrer 130 was rotated at 50 to 500 rpm.

### Embodiment 1-2

The solvent 1101 was contained in the batch reactor 1200 of FIG. 2 , and hydrogen chloride, which is the raw material 1102, was supplied to the solvent 1101 by the sparger 120 to produce xylylene diisocyanate. In the Embodiment 1-2, one stage of axial-type impeller 1522 and one stage of radial-type impeller 1521 were used. The stirrer 150 was rotated at 50 to 500 rpm.

### Embodiment 1-3

The solvent 1101 was contained in the batch reactor 1500 of FIG. 5 , and hydrogen chloride, which is the raw material 1102, was supplied to the solvent 1101 by the sparger 120 to produce xylylene diisocyanate. In the Embodiment 1-3, one stage of radial-type impeller 1321 and four stages of axial-type impeller 1322 were used. The stirrer 130 was rotated at 50 to 500 rpm.

### Embodiment 1-4

The solvent 1101 was contained in the batch reactor 1600 of FIG. 6, and hydrogen chloride, which is the raw material 1102, was supplied to the solvent 1101 by the sparger 120 to produce xylylene diisocyanate. In the Embodiment 1-4, one stage of axial-type impeller 1522 and four stages of radial-type impeller 1521 were used. The stirrer 150 was rotated at 50 to 500 rpm.

### Embodiment 1-5

The solvent 1101 was contained in the batch reactor 1700 of FIG. 7 , and hydrogen chloride, which is the raw material 1102, was supplied to the solvent 1101 by the sparger 120 to produce xylylene diisocyanate. In Embodiment 1-5, the three stages of axial-type impellers 1522 and the two stages of radial-type impellers 1521 were used, with a total of five impellers installed alternately. The stirrer 150 was rotated at 50 to 500 rpm.

### Comparative Example 1-1

The solvent 1101 was contained in the batch reactor 1300 of FIG. 3 , and hydrogen chloride, which is the raw material 1102, was supplied to the solvent 1101 by the sparger 120 to produce xylylene diisocyanate. In Comparative Example 1-1, two-stages of radial-type impeller 162 were used. The stirrer 160 was rotated at 50 to 500 rpm.

### Comparative Example 1-2

The solvent 1101 was contained in the batch reactor 1400 of FIG. 4 , and hydrogen chloride, which is the raw material 1102, was supplied to the solvent 1101 by the sparger 120 to produce xylylene diisocyanate. In Comparative Example 1-2, two stages of axial-type impeller 172 were used. The stirrer 170 was rotated at 50 to 500 rpm.

### Experimental Example 1

The gas holdups of Embodiments 1-1 to 1-5, Comparative Example 1-1, and Comparative Example 1-2 were measured. In Embodiment 1-1, the gas holdup exceeds 10 % for a reaction time of 6.0 hours, in Embodiment 1-2, the gas holdup exceeds 10 % for a reaction time of 6.3 hours, in Embodiment 1-3, the gas holdup exceeds 10 % for a reaction time of 4.5 hours, and in Embodiment 1-4, the gas holdup exceeds 10 % for a reaction time of 5.3 hours. in Embodiment 1-5, the gas holdup exceeds 10 % for a reaction time of 5.2 hours, whereas in Comparative Example 1-1, the gas holdup is 4.7 % for a reaction time of 7.8 hours, and in Comparative Example 1-2, the gas holdup is 8.0 % for a reaction time of 6.7 hours.

That is, Embodiments 1-1 to 1-5 exhibit an increased gas holdup despite a shorter reaction time compared to Comparative Examples 1-1 and 1-2. Therefore, compared to Comparative Examples 1-1 and 1-2, Embodiments 1-1 to 1-5 can improve reactivity of aliphatic isocyanate using hydrogen chloride by inducing a conformational change in hydrogen chloride that allows hydrogen chloride, which is the raw material 1102 and participating in the reaction, to participate efficiently in the reaction.

Hereinafter, a second embodiment of the present disclosure will be described.

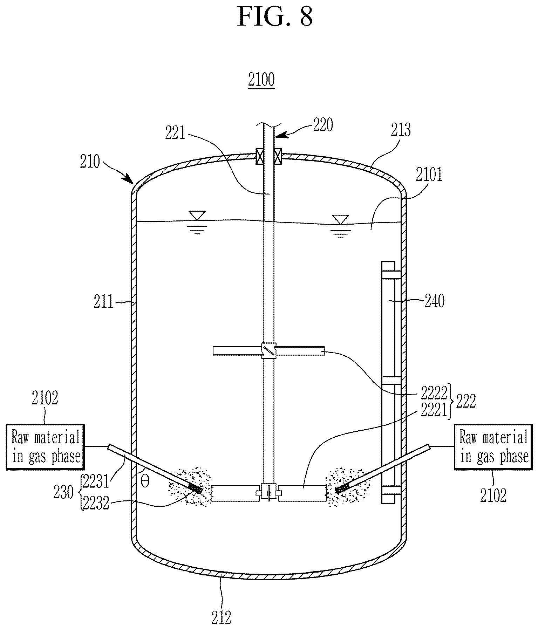

FIG. 8 is a cross-sectional view of a batch reactor according to Embodiment 2-1 of the present disclosure. With reference to FIG. 8, a batch reactor 2100 of Embodiment 2-1 includes a reactor 210 that receives a solvent 2101 in a liquid phase, a stirrer 220, and a sparger 230 that supplies a raw material 2102 in a gas phase into the reactor 210.

For an example, the reactor 210 includes a cylindrical sidewall portion 211 and a bottom portion 212 and a cover portion 213 that form lower and upper sides of the sidewall portion 211, respectively, to form a reaction space. The bottom portion 212 and the cover portion 213 are formed with convex curved surfaces on the lower and upper sides, so that the solvent 2101 in a liquid phase and the raw material 2102 in a gas phase may flow and be effectively mixed.

The stirrer 220 includes a rotational shaft 221 installed in the reactor 210 in a height direction, and an impeller 222 provided on the rotational shaft rotational shaft 221 to stir the solvent 2101 and the raw material 2102. The rotational shaft 221 is rotatably installed on the cover portion 213 of the reactor 210, a lower end thereof faces the bottom portion 212, and may be driven by an externally provided drive motor (not illustrated).

For an example, the impeller 222 includes a radial-type impeller 2221 and an axial-type impeller 2222. The radial-type impeller 2221 generates flow in a transverse direction perpendicular to the rotational shaft 221 to crush and disperse the raw material 2102 in the gas phase supplied in the solvent 2101 in the liquid phase. The axial-type impeller 2222 generates flow in the same axial direction as the rotational shaft 221 to crush and disperse the raw material 2102 in the gas phase supplied in the solvent 2101 in the liquid phase.

In Embodiment 2-1, the radial-type impeller 2221 is provided in one stage on a lower end of the rotational shaft 221, and the axial-type impeller 2222 may be provided in one stage on the rotational shaft 221 on an upper side of the radial-type impeller 2221. Although not illustrated more, the radial-type impeller 2221 and the axial-type impeller 2222 may be installed in one or more stages depending on heights of the rotational shaft 221 and the reactor 210.

In the stirrer of the present disclosure, the impeller may be installed with a radial-type impeller or an axial-type impeller at the lower end and one, two or more, or a plurality of impellers of the same or different types may be provided at the top of the impeller installed at the lower end. In addition, in the stirrer, the impellers may be provided alternately up to the n^{th} stage, which requires a radial-type impeller and an axial-type impeller. The various drawings corresponding to these cases are omitted.

The spargers 230 are formed and installed to supply the raw material 2102 in the gas phase in at least one direction of an axial direction of the stirrer 220 and a circumferential direction of the reactor 210. The sparger 230 may be provided in plural.

According to Embodiment 2-1, two spargers 230 are installed on the sidewall portion 211 of the reactor 210 at a predetermined interval along the circumferential direction of the stirrer 220.

The sparger 230 includes a pipe 231 and a bubble formation portion 232. The pipe 231 is installed in the sidewall portion 211 of the reactor 210 in a direction in which a length direction thereof intersects the axial direction of the stirrer 220. For an example, the pipe 231 is installed in the sidewall portion 211 at a predetermined angle θ.

The bubble formation portion 232 is provided with a plurality of spray ports at an end of the pipe 231 to supply the raw material 2102 in the gas phase in a bubble state toward the impeller 222. A plurality of spargers 230 are provided in the reactor 210 along the circumferential direction to enable uniform distribution of the raw material 2102 in the gas phase in the circumferential direction.

The sparger 230 may be installed corresponding to the impeller 222. Therefore, the impeller 222 may distribute the raw material 2102 in the gas phase supplied by the sparger 230 uniformly within the interval, further uniformly distributing the raw material 2102 in the gas phase, which is hydrogen chloride (HCl), throughout the circumferential direction of the reactor 210.

Meanwhile, the radial-type impeller 2221 is rotationally driven around the raw material 2102 in the gas phase supplied from the bubble formation portion 232 of the sparger 230 to flow the solvent 2101 in the transverse direction. Therefore, the raw material 2102 in the gas phase supplied to the bubble formation portion 232 of the sparger 230 is mixed in the solvent 2101 while flowing in the transverse direction by the transverse flow of the solvent 2101.

The axial-type impeller 2222 is driven on an upper side of the radial-type impeller 2221 to flow the solvent 2101 in the axial direction. Therefore, the partially mixed raw material 2102 in the gas phase and the raw material 2102 in the gas phase are further mixed in the solvent 2101 while flowing in the axial direction by the axial flow of the solvent 2101.

As the salt formation reaction proceeds by mixing the raw material 2102 in the solvent 2101, amine salts are produced. The amine salts, which are products, are in the form of particles with a diameter of several to tens of micrometers, so as the reaction proceeds, the amine salts change into the form of a slurry that is dispersed in the solvent.

As described above, the plurality of spargers 230 disposed in the circumferential direction uniformly distribute and supply the raw material 2102 in the gas phase to the solvent 2101, thereby increasing the gas holdup within a certain period of time. Therefore, a reaction rate within the reactor 210 can be increased. In conclusion, distribution performance of hydrogen chloride to the solvent can be improved in a reaction in which hydrogen chloride, which is the raw material 2102, is supplied to the solvent 2101 to produce a slurry state. Therefore, a reaction rate within the reactor 210 can be increased.

Meanwhile, the reactor 210 further includes a baffle 240 disposed in parallel with the rotational shaft 221 and installed on an inner surface, that is, an inner side of the sidewall portion 211. For example, the baffle 240 may be formed in the form of a plate having a predetermined width and height, or may be formed in the form of a rod having a predetermined diameter and length.

The baffle 240 creates a vortex in the flowing solvent 2101 when the impeller 222 is driven, allowing for more uniform mixing of the solvent 2101 and the raw material 2102 over the entire area of the reactor 210 in the height direction.

The baffles 240 are provided in plural along a circumferential direction inside the reactor 210 to repeatedly form vortices in the circumferential direction to allow for more uniform mixing of the solvent 2101 and raw material 2102 over the entire area in the circumferential direction.

In addition, the baffle 240 may be provided in a vortex area generated by the rotational drive of the radial-type impeller 2221 and the axial-type impeller 2222, which may directly act on the mixing of the solvent 2101 and the raw material 2102.

In addition to the sparger 230, the use of the radial-type impeller 2221 and the axial-type impeller 2222 and the baffle 240 may further increase the gas holdup when the raw material 2102 in the gas phase is sprayed and mixed in solvent 2101. Therefore, the reaction rate within the reactor 210 may be further increased.

The batch reactor 2100 according to Embodiment 2-1 of the present disclosure is used for a reaction between a gas phase and a liquid phase, and can be used in a process to produce xylylene diisocyanate by supplying hydrogen chloride, which is the raw material 2102 in the gas phase, to a solvent 2101 in the liquid phase.

Hereinafter, various variation examples and comparative examples of the present embodiment are described below. The descriptions of configurations that are identical to Embodiment 2-1 are omitted, and the descriptions of configurations that are different are described.

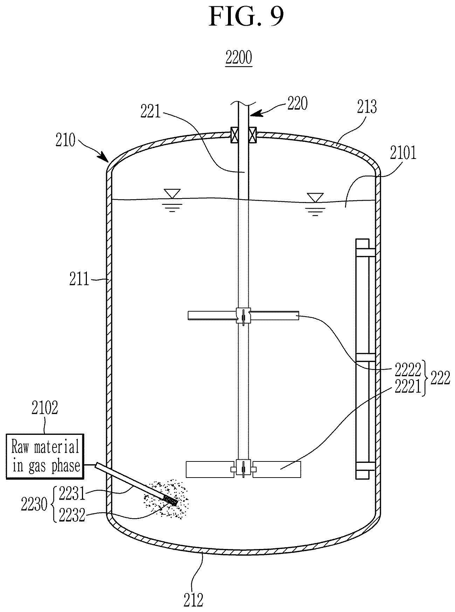

FIG. 9 is a cross-sectional view of a batch reactor according to Embodiment 2-2 of the present disclosure. With reference to FIG. 9, in a batch reactor 2200 of Embodiment 2-2, a single sparger 2230 is provided and installed in the sidewall portion 211 of the reactor 210 at the lower end of the reactor 210.

The sparger 2230 includes a pipe 231 and a bubble formation portion 232. The pipe 231 is installed in the sidewall portion 211 of the reactor 210 in a direction in which a length direction thereof intersects the axial direction of the stirrer 220. The bubble formation portion 232 is provided with a plurality of spray ports at the end of the pipe 231 to supply the raw material in the gas phase in a bubble state to the lower end of the reactor 210 toward a downstream of the impeller.

Meanwhile, the impeller 222 is rotationally driven at the upper side of the raw material 2102 in the gas phase supplied from the bubble formation portion 232 of the sparger 2230 to flow the solvent 2101. Therefore, the raw material 2102 in the gas phase supplied to the bubble formation portion 232 of the sparger 2230 is mixed in the solvent 2101 while flowing by the flow of the solvent 2101.

As described above, the single sparger 2230 supplies the raw material 2102 in the gas phase to the solvent 2101, thereby increasing the gas holdup within a certain period of time. Therefore, a reaction rate within the reactor 210 can be increased. In conclusion, distribution performance of hydrogen chloride to the solvent can be improved in a reaction in which hydrogen chloride, which is the raw material 2102, is supplied to the solvent 2101 to produce a slurry state. Therefore, a reaction rate within the reactor 210 can be increased.

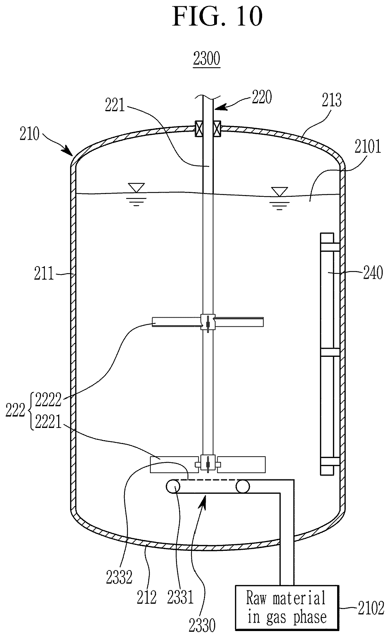

FIG. 10 is a cross-sectional view of a batch reactor according to Embodiment 2-3 of the present disclosure. With reference to FIG. 10, in the batch reactor 2300 of Embodiments 2-3, the sparger 2330 includes a ring 2331 and a bubble formation portion 2332. The ring 2331 is provided in a circular shape on the lower side of the stirrer 220, and the bubble formation portion 2332 is provided on an upper side of the ring 2331 to supply the raw material in the gas phase in a bubble state toward a downstream side of the impeller 222. The sparger 2330 enables uniform distribution of the raw material in the gas phase in the circumferential direction according to the diameter of the ring 2331.

Meanwhile, the impeller 222 is rotationally driven at the upper side of the raw material 2102 in the gas phase supplied from the bubble formation portion 2332 of the sparger 2330 to flow the solvent 2101. Therefore, the raw material 2102 in the gas phase supplied to the bubble formation portion 2332 of the sparger 2330 is mixed in the solvent 2101 while flowing by the flow of the solvent 2101.

As described above, the sparger 2330, which is disposed in a circular shape on the lower side of the stirrer 220, uniformly distributes and supplies the raw material 2102 in the gas phase to the solvent 2101 in the circumferential direction, thereby increasing the gas holdup within a certain time. Therefore, a reaction rate within the reactor 210 can be increased. In conclusion, distribution performance of hydrogen chloride to the solvent can be improved in a reaction in which hydrogen chloride, which is the raw material 2102, is supplied to the solvent 2101 to produce a slurry state. Therefore, a reaction rate within the reactor 210 can be increased.

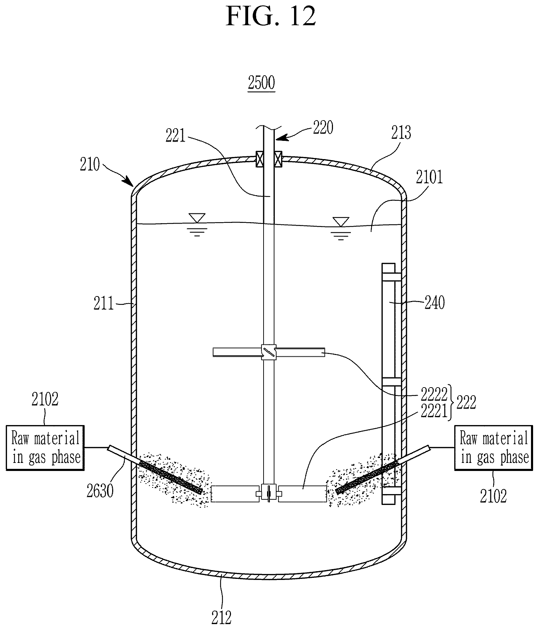

FIG. 12 is a cross-sectional view of a batch reactor according to Embodiments 2-4 of the present disclosure. With reference to FIG. 12, in a batch reactor 2500 of Embodiment 2-4, two spargers 2630 are provided and installed in the sidewall portion 211 of the reactor 210 at the lower end of the reactor 210.

The sparger 2630 is formed as a sintered porous type. The sintered porous type sparger 2630 forms porosity over the entire length within the reactor 210 to allow for uniform distribution of the raw material 2102 in the gas phase in the circumferential direction.

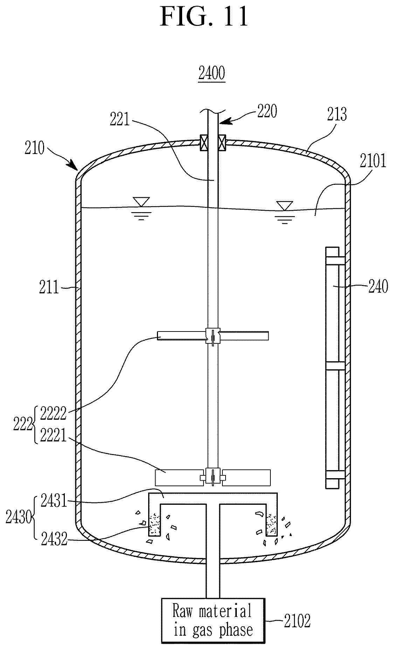

FIG. 11 is a cross-sectional view of a batch reactor according to Comparative Example 2. With reference to FIG. 11, in a batch reactor 2400 of Comparative Example 2, a sparger 2430 is formed as a spider type and includes a plurality of branches 2431 and a bubble formation portion 2432.

A plurality of branches 2431 are provided on the lower side of the stirrer 220, and the bubble formation portion 2432 is provided on a lower side of the branches 2431 to supply the raw material in the gas phase in a bubble state toward the bottom portion 212. The sparger 2430 supplies the raw material in the gas phase to a far side of the stirrer 220 in the axial direction along a length of the branch 2431.

Meanwhile, the impeller 222 is rotationally driven at the upper side of the raw material 2102 in the gas phase supplied from the bubble formation portion 2432 of the sparger 2430 to flow the solvent 2101. Therefore, the raw material 2102 in the gas phase supplied to the bubble formation portion 2432 of the sparger 2430 is mixed in the solvent 2101 while flowing by the flow of the solvent 2101. Compared to Embodiments 2-1, 2-2, and 2-3, it is difficult to improve distribution performance of hydrogen chloride to the solvent 2101 in the comparative example.

### Table

Table 2 shows the gas holdup for Embodiment 2-1, Embodiment 2-2, Embodiment 2-3, and Comparative Example 2.

**(Table 2)**

| | Embodiment 2-1 | Embodiment 2-2 | Embodiment 2-3 | Embodiment 2-4 | Comparative Example2 |
|---|---|---|---|---|---|
| Type | Circumferentially spaced pipes | Pipes | Rings | Sintered porous type | Spider |
| Number | 2 | 1 | - | 2 | - |
| Gas holdup (%) | >10 | >10 | >10 | >10 | >5 |
| Reaction time (hr) | 4.8 | 5.5 | 4.8 | 4.8 | 7.1 |

### Embodiment 2-1

The solvent 2101 was contained in the batch reactor 2100 of FIG. 8 , and hydrogen chloride, which is the raw material 2102, was supplied to the solvent 2101 by the sparger 230 to produce xylylene diisocyanate. In Embodiment 2-1, two pipe type spargers 230 are used. The stirrer 220 was rotated at 50 to 500 rpm.

### Embodiment 2-2

The solvent 2101 was contained in the batch reactor 2200 of FIG. 9 , and hydrogen chloride, which is the raw material 2102, was supplied to the solvent 2101 by the sparger 2230 to produce xylylene diisocyanate. In Embodiment 2-2, one pipe type sparger 2230 is used. The stirrer 220 was rotated at 50 to 500 rpm.

### Embodiment 2-3

The solvent 2101 was contained in the batch reactor 2300 of FIG. 10 , and hydrogen chloride, which is the raw material 2102, was supplied to the solvent 2101 by the sparger 2330 to produce xylylene diisocyanate. In Embodiment 2-3, one ring type sparger 2330 is used. The stirrer 220 was rotated at 50 to 500 rpm.

### Embodiment 2-4

The solvent 2101 was contained in the batch reactor 2500 of FIG. 12 , and hydrogen chloride, which is the raw material 2102, was supplied to the solvent 2101 by the sparger 2630 to produce xylylene diisocyanate. In Embodiments 2-4, two sintered porous type spargers 2630 are used. The stirrer 220 was rotated at 50 to 500 rpm.

### Comparative Example2

The solvent 2101 was contained in the batch reactor 2400 of FIG. 11 , and hydrogen chloride, which is the raw material 2102, was supplied to the solvent 2101 by the sparger 2430 to produce xylylene diisocyanate. In the Comparative Example, a spider type sparger 2430 is used. The stirrer 220 was rotated at 50 to 500 rpm.

### Experimental Example 2

The gas holdups of Embodiment 2-1, Embodiment 2-2, Embodiment 2-3, and Comparative Example 2 were measured. In Embodiment 2-1, the gas holdup exceeds 10 % for a reaction time of 4.8 hours, in Embodiment 2-2, the gas holdup exceeds 10 % for a reaction time of 5.5 hours, in Embodiment 2-3, the gas holdup exceeds 10 % for a reaction time of 4.8 hours, and in Embodiment 2-4, the gas holdup exceeds 10 % for a reaction time of 4.8 hours, whereas in Comparative Example 2, the gas holdup exceeds 5 % for a reaction time of 7.1 hours.

That is, Embodiments 2-1, 2-2, 2-3, and 2-4 exhibit an increased gas holdup despite a shorter reaction time compared to Comparative Example 2. Therefore, compared to Comparative Example 2, Embodiments 2-1, 2-2, 2-3, and 2-4 may increase the amount of hydrogen chloride, which is the raw material 2102 participating in the reaction, to improve reactivity of the aliphatic isocyanate using hydrogen chloride.

Hereinafter, a third embodiment of the present disclosure will be described.

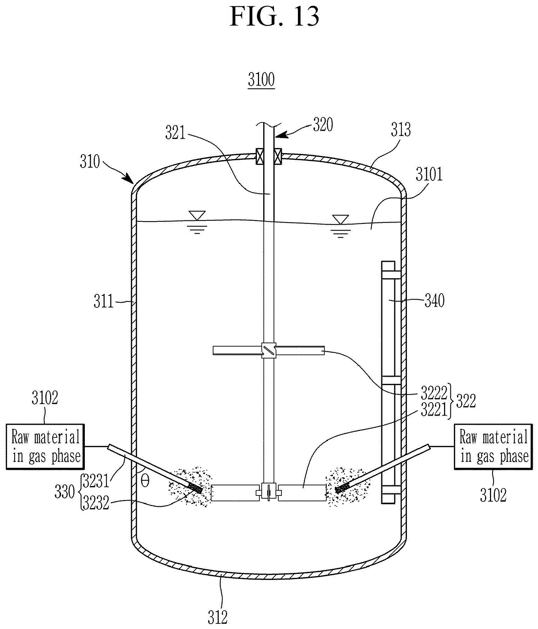

FIG. 13 is a cross-sectional view of a batch reactor according to Embodiment 3-1 of the present disclosure. With reference to FIG. 13, a batch reactor 3100 of Embodiment 3-1 includes a reactor 310 that receives amine salts 3101 in a slurry phase, a sparger 320 that supplies a raw material 3102 in a gas phase into the reactor 310, and a stirrer 330.

For an example, the reactor 310 includes a cylindrical sidewall portion 31 1 and a bottom portion 312 and a cover portion 313 that form lower and upper sides of the sidewall portion 311, respectively, to form a reaction space. The bottom portion 312 and the cover portion 313 are formed with convex curved surfaces on the lower and upper sides, so that the amine salts 3101 in the slurry phase and the raw material 3102 in a gas phase may flow and be effectively mixed.

The spargers 320 are formed and installed to supply the raw material 3102 in the gas phase in the axial direction of the stirrer 330 and the circumferential direction of the reactor 310. The sparger 320 includes a pipe 321 and a bubble formation portion 322. The pipe 321 is installed in the sidewall portion 311 of the reactor 310 in a direction in which a length direction thereof intersects the axial direction of the stirrer 330. For an example, the pipe 321 is installed in the sidewall portion 311 at a predetermined angle θ.

The bubble formation portion 322 is provided with a plurality of spray ports at an end of the pipe 321 to supply the raw material 3102 in the gas phase in a bubble state toward the impeller 332. In the sparger 320, the plurality of bubble formation portions 322 enable uniform spraying of phosgene, which is a raw material 3102 in the gas phase, into the amine salts 3101 in the slurry phase.

The stirrer 330 includes a rotational shaft 331 installed in the reactor 310 in a height direction, and an impeller 332 provided on the rotational shaft rotational shaft 331 to stir the amine salts 3101 and the raw material 3102. The impeller 332 is disposed in a plurality of stages on the rotational shaft 331. The rotational shaft 331 is rotatably installed on the cover portion 313 of the reactor 310, a lower end thereof faces the bottom portion 312, and may be driven by an externally provided drive motor (not illustrated).

The impeller 332 may be formed as at least one of a radial-type impeller and an axial-type impeller. In Embodiment 3-1, the impeller 332 includes a radial-type impeller 3321 and an axial-type impeller 3322.

The radial-type impeller 3321 generates flow in a transverse direction perpendicular to the rotational shaft 331 to crush and disperse the raw material 3102 in the gas phase supplied in the amine salts 3101 in the slurry phase. The axial-type impeller 3322 generates flow in the same axial direction as the rotational shaft 331 to crush and disperse the raw material 3102 in the gas phase supplied in the amine salts 3101 in the slurry phase.

In Embodiment 3-1, the radial-type impeller 3321 is provided in one stage on a lower end of the rotational shaft 331, and the axial-type impeller 3322 may be provided in four stages on the rotational shaft 331 on an upper side of the radial-type impeller 3321. That is, the radial-type impeller 3321 is installed on a first stage, and the axial-type impeller 3322 is installed on a second stage to a fifth stage.

In the stirrer of the present disclosure, the impeller may be installed with a radial-type impeller or an axial-type impeller at the lower end and one, two or more, or a plurality of impellers of the same or different types may be provided at the top of the impeller installed at the lower end. In addition, in the stirrer, the impellers may be provided alternately up to the n^{th} stage, which requires a radial-type impeller and an axial-type impeller. The various drawings corresponding to these cases are omitted.

In Embodiment 3-1, the radial-type impeller 3321 is rotationally driven around the raw material 3102 in the gas phase supplied from the bubble formation portion 322 of the sparger 320 to flow the amine salts 3101 in the transverse direction. Therefore, the raw material 3102 in the gas phase sprayed to the bubble formation portion 322 of the sparger 320 is mixed in the amine salts 3101 while flowing in the transverse direction by the transverse flow of the amine salts 3101.

The four stages of axial-type impeller 3322 is driven on an upper side of the radial-type impeller 3321 to flow the amine salts 3101 in the axial direction. Therefore, the partially mixed raw material 3102 in the gas phase and the raw material 3102 in the gas phase are further mixed in the amine salts 3101 while flowing in the axial direction by the axial flow of the amine salts 3101.

As described above, the mixed use of one stage of the radial-type impeller 3321 and four stages of the axial-type impeller 3322 can increase the gas holdup when the raw material 3102 in the gas phase is sprayed and mixed in the amine salts 3101. In conclusion, distribution performance of phosgene to the reactants in the slurry state can be improved in an ongoing phosgenation reaction in a state in which phosgene, which is the raw material 3102, is supplied to the amine salts 3101. Therefore, a reaction rate within the reactor 310 can be increased.

Meanwhile, the reactor 310 further includes a baffle 340 disposed in parallel with the rotational shaft 331 and installed on an inner surface, that is, an inner side of the sidewall portion 311. For example, the baffle 340 may be formed in the form of a plate having a predetermined width and height, or may be formed in the form of a rod having a predetermined diameter and length.

The baffle 340 creates a vortex in the flowing amine salts 3101 when the impeller 332 is driven, allowing for more uniform mixing of the amine salts 3101 and the raw material 3102 over the entire area of the reactor 310 in the height direction.

The baffles 340 are provided in plural along a circumferential direction inside the reactor 310 to repeatedly form vortices in the circumferential direction to allow for more uniform mixing of the amine salts 3101 and raw material 3102 over the entire area in the circumferential direction.

In addition, the baffle 340 may be provided in a vortex area generated by the rotational drive of the radial-type impeller 3321 and the axial-type impeller 3322, which may directly act on the mixing of the amine salts 3101 and the raw material 3102.

The use of the radial-type impeller 3321 and the axial-type impeller 3322 and the baffle 340 may further increase the gas holdup when the raw material 3102 in the gas phase is sprayed and mixed in the amine salts 3101. Therefore, the reaction rate within the reactor 310 may be further increased.

The batch reactor 3100 according to Embodiment 3-1 of the present disclosure is used for a reaction between a gas phase and a slurry phase, and can be used in a process to produce xylylene diisocyanate by supplying phosgene, which is the raw material 3102 in the gas phase, to the amine salts 3101 in the slurry phase.

Hereinafter, various variation examples and comparative examples of the present embodiment are described below. The descriptions of configurations that are identical to Embodiment 3-1 are omitted, and the descriptions of configurations that are different are described.

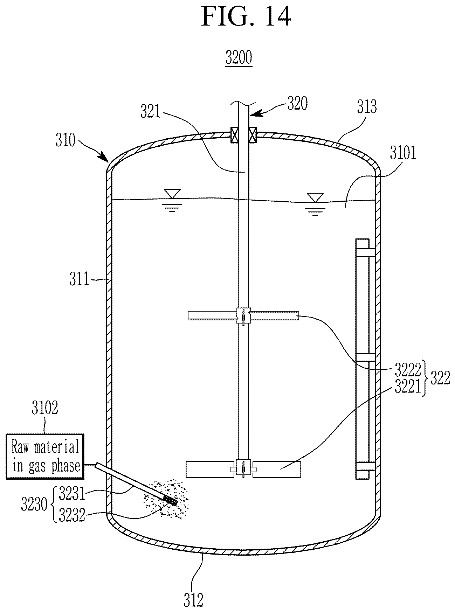

FIG. 14 is a cross-sectional view of a batch reactor according to Embodiment 3-2 of the present disclosure. With reference to FIG. 14, the impeller 332 of a batch reactor 3200 of Embodiment 3-2 is formed in two stages. A radial-type impeller 3321 is provided in one stage on a lower end of the rotational shaft 331, and an axial-type impeller 3322 may be provided in one stage on the rotational shaft 331 on an upper side of the radial-type impeller 3321. That is, the radial-type impeller 3321 is installed on a first stage, and the axial-type impeller 3322 is installed on a second stage.

Although not separately illustrated, an axial-type impeller may be provided in one stage at the lower end of the rotating shaft, and a radial-type impeller may be provided in one stage on the rotating shaft at the upper side of the axial-type impeller.

In Embodiment 3-2, the radial-type impeller 3321 is rotationally driven at the upper side of the raw material 3102 in the gas phase sprayed from the spray ports 321 of the sparger 320 to flow the amine salts 3101 in the transverse direction. Therefore, the raw material 3102 in the gas phase sprayed to the spray ports 321 of the sparger 320 is mixed in the amine salts 3101 while flowing in the transverse direction by the transverse flow of the amine salts 3101.

One stage of axial-type impeller 3322 is driven on an upper side of the radial-type impeller 3321 to flow the amine salts 3101 in the axial direction. Therefore, the partially mixed raw material 3102 in the gas phase and the raw material 3102 in the gas phase are further mixed in the amine salts 3101 while flowing in the axial direction by the axial flow of the amine salts 3101.

As described above, the mixed use of one stage of radial-type impeller 3321 and one stage of axial-type impeller 3322 can increase the gas holdup when the raw material 3102 in the gas phase is sprayed and mixed in the amine salts 3101. In conclusion, distribution performance of phosgene to the reactants in the slurry state can be improved in an ongoing phosgenation reaction in which phosgene, which is the raw material 3102, is supplied to the amine salts 3101. Therefore, a reaction rate within the reactor 310 can be increased.

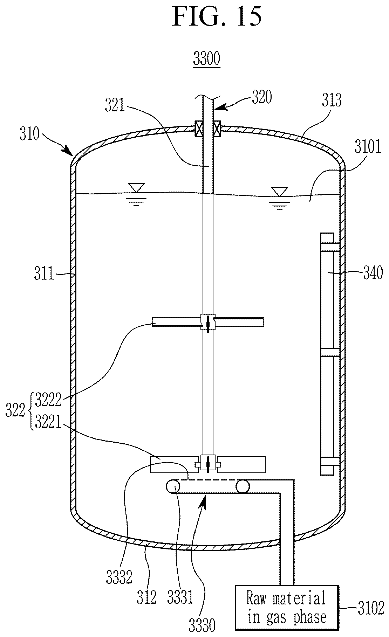

FIG. 15 is a cross-sectional view of a batch reactor according to Comparative Example 3. With reference to FIG. 15, in a batch reactor 3300 of Comparative Example 3, a stirrer 3330 has one radial-type impeller 3323 mounted on the rotational shaft 331.

The radial-type impeller 3323 is rotationally driven on the upper side of the raw material 3102 in the gas phase sprayed from the sparger 320 to flow the amine salts 3101 in the transverse direction. Therefore, the raw material 3102 in the gas phase sprayed to the spray ports 321 of the sparger 320 is mixed in the amine salts 3101 while flowing in the circumferential direction by the circumferential flow of the amine salts 3101.

Compared to Embodiments 3-1 and 3-2, Comparative Example 3 only forms a flow in one transverse direction with respect to the amine salts 3101, thereby making it difficult to form an axial flow.

### Table

Table 1 shows the gas holdup for Embodiment 3-1, Embodiment 3-2, and Comparative Example 3.

**(Table 3)**

| | Embodiment 3-1 | Embodiment 3-2 | Comparative Example 3 |
|---|---|---|---|
| Stage number of impeller | >2 | 2 | 1 |
| Gas holdup (%) | >10 | >10 | 4.7 |
| Reaction time (hr) | 4.5 | 5.2 | 7.8 |

### Embodiment 3-1

The amine salts 3101 were contained in the batch reactor 3100 of FIG. 13 , and phosgene, which is the raw material 3102, was supplied to the amine salts 3101 by the sparger 320 to produce xylylene diisocyanate. In Embodiment 3-1, five stages of the impeller 332 are used. The stirrer 330 was rotated at 50 to 500 rpm.

### Embodiment 3-2

The amine salts 3101 were contained in the batch reactor 3200 of FIG. 14 , and phosgene, which is the raw material 3102, was supplied to the amine salts 3101 by the sparger 320 to produce xylylene diisocyanate. In Embodiment 3-2, two stages of the impeller 332 are used. The stirrer 3230 was rotated at 50 to 500 rpm.

### Comparative Examples

The amine salts 3101 were contained in the batch reactor 3300 of FIG. 15 , and phosgene, which is the raw material 3102, was supplied to the amine salts 3101 by the sparger 320 to produce xylylene diisocyanate. In the comparative example, one stage of the radial-type impeller 3323 is used. The stirrer 3330 was rotated at 50 to 500 rpm.

### Experimental Example 3

The gas holdups of Embodiment 3-1, Embodiment 3-2, and Comparative Example 3 were measured. In Embodiment 3-1, the gas holdup exceeded 10 % for a reaction time of 4.5 hours, and in Embodiment 3-2, the gas holdup exceeded 5.2 % for a reaction time of 5.2 hours, whereas in Comparative Example 3, the gas holdup was 4.7 % for a reaction time of 7.8 hours.

That is, Embodiment 3-1 and Embodiment 3-2 exhibit an increased gas holdup despite a shorter reaction time compared to Comparative Examples 3. Therefore, compared to Comparative Example 3, Embodiment 3-1 and Embodiment 3-2 may increase the amount of phosgene, which is the raw material 3102 participating in the reaction, to improve reactivity of the aliphatic isocyanate using phosgene.

Hereinafter, a fourth embodiment of the present disclosure will be described.

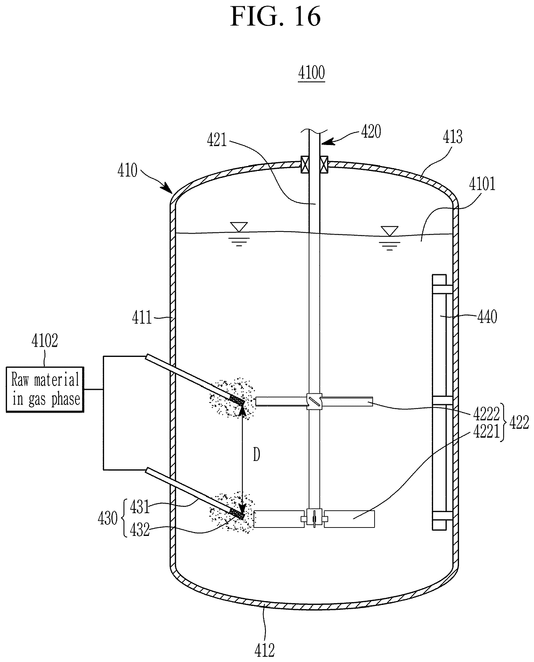

FIG. 16 is a cross-sectional view of a batch reactor according to Embodiment 4-1 of the present disclosure. With reference to FIG. 16, a batch reactor 4100 of Embodiment 4-1 includes a reactor 410 that receives amine salts 4101 in a slurry phase produced in a first step salt formation reaction, a stirrer 420, and a sparger 430 that supplies a raw material 4102 in a gas phase into the reactor 410.

For an example, the reactor 410 includes a sidewall portion 411 in the shape of a cylindrical and a bottom portion 412 and a cover portion 413 that form lower and upper sides of the sidewall portion 411, respectively, to form a reaction space. The bottom portion 412 and the cover portion 413 are formed with convex curved surfaces on the lower and upper sides, so that the amine salts 4101 in the slurry phase and the raw material 4102 in a gas phase may flow and be effectively mixed.

The stirrer 420 includes a rotational shaft 421 installed in the reactor 410 in a height direction, and an impeller 422 provided on the rotational shaft rotational shaft 421 to stir the amine salts 4101 and the raw material 4102. The rotational shaft 421 is rotatably installed on the cover portion 413 of the reactor 410, a lower end thereof faces the bottom portion 412, and may be driven by an externally provided drive motor (not illustrated).

For an example, the impeller 422 includes a radial-type impeller 4221 and an axial-type impeller 4222. The radial-type impeller 4221 generates flow in a transverse direction perpendicular to the rotational shaft 421 to crush and disperse the raw material 4102 in the gas phase supplied in the amine salts 4101. The axial-type impeller 4222 generates flow in the same axial direction as the rotational shaft 421 to crush and disperse the raw material 4102 in the gas phase supplied in the amine salts 4101.

In Embodiment 4-1, the radial-type impeller 4221 is provided in one stage on a lower end of the rotational shaft 421, and the axial-type impeller 4222 may be provided in one stage on the rotational shaft 421 on an upper side of the radial-type impeller 4221. Although not illustrated more, the axial-type impeller 4222 may also be installed in a greater number of stages depending on heights of the rotational shaft 421 and the reactor 410.

The spargers 430 are formed and installed in plural to supply the raw material 4102 in the gas phase in one direction of an axial direction of the stirrer 420 and a circumferential direction of the reactor 410. According to Embodiment 4-1, the plurality of spargers 430 are installed on the sidewall portion 411 of the reactor 410 at a predetermined interval D along the axial direction of the stirrer 420.

The sparger 430 includes a pipe 431 and a bubble formation portion 432. The pipe 431 is installed in the sidewall portion 411 of the reactor 410 in a direction in which a length direction thereof intersects the axial direction of the stirrer 420. For an example, the pipe 431 is installed in the sidewall portion 411 at a predetermined angle θ.

The bubble formation portion 432 is provided with a plurality of spray ports at an end of the pipe 431 to supply the raw material 4102 in the gas phase in a bubble state toward the impeller 422. A plurality of spargers 430 are provided in the reactor 10 along the axial direction to enable uniform distribution of the raw material 4102 in the gas phase in the axial direction.

When the impeller 422 is provided in plural, the sparger 430 may be installed corresponding to the impeller 422. Therefore, each impeller 422 may distribute the raw material 4102 in the gas phase supplied by each sparger 430 uniformly within the interval D, further uniformly distributing the raw material 4102 in the gas phase throughout the axial direction of the reactor 410.

Meanwhile, the radial-type impeller 4221 is rotationally driven around the raw material 4102 in the gas phase supplied from the bubble formation portion 432 of the sparger 430 to flow the amine salts 4101 in the transverse direction. Therefore, the raw material 4102 in the gas phase supplied to the bubble formation portion 432 of the sparger 430 is mixed in the amine salts 4101 while flowing in the transverse direction by the transverse flow of the amine salts 4101.

The axial-type impeller 4222 is driven on an upper side of the radial-type impeller 4221 to flow the amine salts 4101 in the axial direction. Therefore, the partially mixed raw material 4102 in the gas phase and the raw material 4102 in the gas phase are further mixed in the amine salts 4101 while flowing in the axial direction by the axial flow of the amine salts 4101.

As described above, the plurality of spargers 430 disposed in the axial direction uniformly distribute and supply the raw material 4102 in the gas phase to the amine salts 4101, thereby increasing the gas holdup within a certain period of time. In conclusion, distribution performance of phosgene to the amine salts in the slurry state can be improved in an ongoing phosgenation reaction in which phosgene, which is the raw material 4102, is supplied to the amine salts 4101. Therefore, a reaction rate within the reactor 410 can be increased.

Meanwhile, the reactor 410 further includes a baffle 440 disposed in parallel with the rotational shaft 421 and installed on an inner surface, that is, an inner side of the sidewall portion 411. For example, the baffle 440 may be formed in the form of a plate having a predetermined width and height, or may be formed in the form of a rod having a predetermined diameter and length.

The baffle 440 creates a vortex in the flowing amine salts 4101 when the impeller 422 is driven, allowing for more uniform mixing of the amine salts 4101 and the raw material 4102 over the entire area of reactor 410 in the height direction.

The baffles 440 are provided in plural along a circumferential direction inside the reactor 410 to repeatedly form vortices in the circumferential direction to allow for more uniform mixing of the amine salts 4101 and raw material 4102 over the entire area in the circumferential direction.

In addition, the baffle 440 may be provided in a vortex area generated by the rotational drive of the radial-type impeller 4221 and the axial-type impeller 4222, which may directly act on the mixing of the amine salts 4101 and the raw material 4102.

In addition to the sparger 430, the use of the radial-type impeller 4221 and the axial-type impeller 4222 and the baffle 440 may further increase the gas holdup when the raw material 4102 in the gas phase is sprayed and mixed in amine salts 4101. Therefore, the reaction rate within the reactor 410 may be further increased.

The batch reactor 4100 according to Embodiment 4-1 of the present disclosure is used for a reaction between a gas phase and a slurry phase, and can be used in a process to produce xylylene diisocyanate by supplying phosgene, which is the raw material 4102 in the gas phase, to the amine salts 4101.

Hereinafter, various variation examples and comparative examples of the present embodiment are described below. The descriptions of configurations that are identical to Embodiment 4-1 are omitted, and the descriptions of configurations that are different are described.

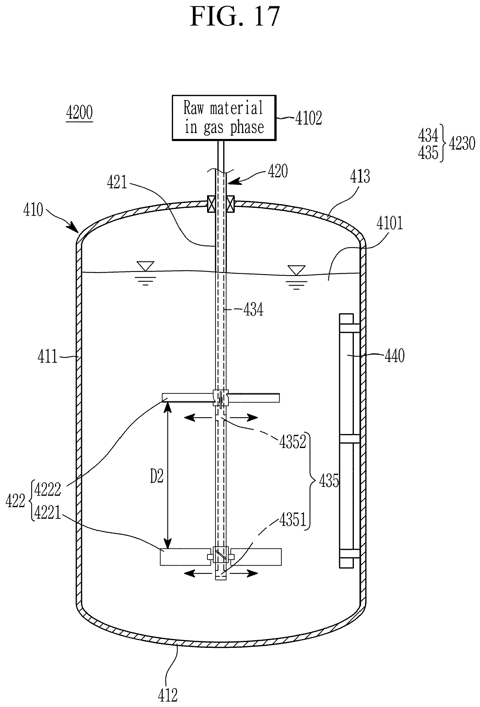

FIG. 17 is a cross-sectional view of a batch reactor according to Embodiment 4-2 of the present disclosure. With reference to FIG. 17, in a batch reactor 4200 of Embodiments 4-2, a sparger 4230 provided in plural includes a guide path 434 and a bubble formation portion 435.

The guide path 434 is penetratively formed within the rotational shaft 421 of the stirrer 420 to supply the raw material 4102 in the gas phase. The bubble formation portion 435 penetrates the rotational shaft 421 and is connected to the guide path 434 to supply the raw material 4102 in the gas phase to the amine salts 4101 in a bubble state.

The impeller 422 is provided in a plurality of stages by maintaining a predetermined interval D2 on the rotational shaft 421, and includes a radial-type impeller 4221 and an axial-type impeller 4222, which are provided sequentially from the lower side. In this case, the bubble formation portion 435 may be formed in plural to correspond to the impeller 422 and the interval D2.

That is, a first bubble formation portion 4351 is provided on the lower side of the radial-type impeller 4221 to supply a fuel 4102 in the gas phase, and a second bubble formation portion 4352 is then provided on the lower side of the axial-type impeller 4222 to supply the fuel 4102 in the gas phase.

The bubble formation portion 435 is provided with a plurality of spray ports on the rotational shaft 421 to supply the raw material 4102 in the gas phase in a bubble state toward the lower side of the impeller 422. In the sparger 4230, the first and second bubble formation portion 4351 and 4352 are provided in plural in the reactor 410 along the axial direction to enable uniform distribution of the raw material 4102 in the gas phase in the axial direction.

As described above, the first and second bubble formation portions 4351 and 4352 of the sparger 4230 disposed in the axial direction uniformly distribute and supply the raw material 4102 in the gas phase to the amine salts 4101, thereby increasing the gas holdup within a certain period of time. In conclusion, distribution performance of phosgene to the amine salts in the slurry state can be improved in an ongoing phosgenation reaction in which phosgene, which is the raw material 4102, is supplied to the amine salts 4101. Therefore, a reaction rate within the reactor 410 can be increased.

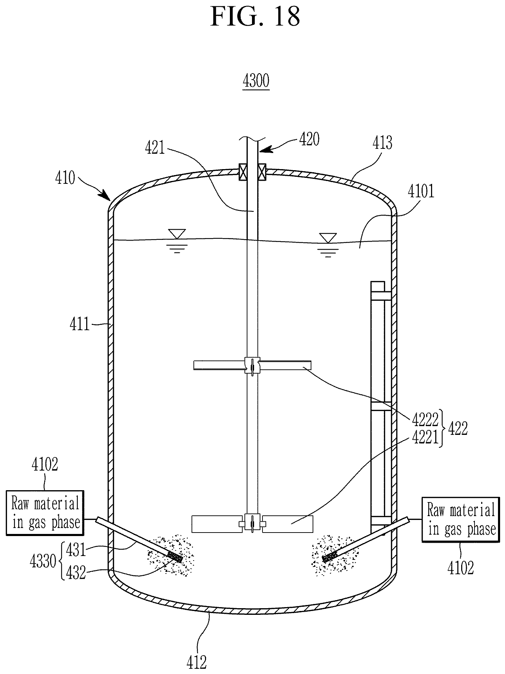

FIG. 18 is a cross-sectional view of a batch reactor according to Embodiment 4-3 of the present disclosure. With reference to FIG. 18, in a batch reactor 4300 of Embodiment 4-3, a plurality of spargers 4330 are provided and installed in the sidewall portion 411 of the reactor 410 at predetermined intervals along the circumferential direction of the reactor 410 at the lower end of the reactor 410.

The sparger 4330 includes a pipe 431 and a bubble formation portion 432. The pipe 431 is installed in the sidewall portion 411 of the reactor 410 in a direction in which a length direction thereof intersects the axial direction of the stirrer 420.

The bubble formation portion 432 is provided with a plurality of spray ports at the end of the pipe 431 to supply the raw material in the gas phase in a bubble state to the lower end of the reactor 410 toward a downstream side of the impeller. A plurality of spargers 4330 are provided in the reactor 410 along the circumferential direction to enable uniform distribution of the raw material in the gas phase in the circumferential direction.

Meanwhile, the impeller 422 is rotationally driven at the upper side of the raw material 4102 in the gas phase supplied from the bubble formation portion 432 of the sparger 4330 to flow the amine salts 4101. Therefore, the raw material 4102 in the gas phase supplied to the bubble formation portion 432 of the sparger 4330 is mixed in the amine salts 4101 while flowing by the flow of the amine salts 4101.

As described above, the plurality of spargers 4330 disposed in the circumferential direction uniformly distribute and supply the raw material 4102 in the gas phase to the amine salts 4101 in the circumferential direction, thereby increasing the gas holdup within a certain period of time. In conclusion, distribution performance of phosgene to the amine salts in the slurry state can be improved in an ongoing phosgenation reaction in which phosgene, which is the raw material 4102, is supplied to the amine salts 4101. Therefore, a reaction rate within the reactor 410 can be increased.

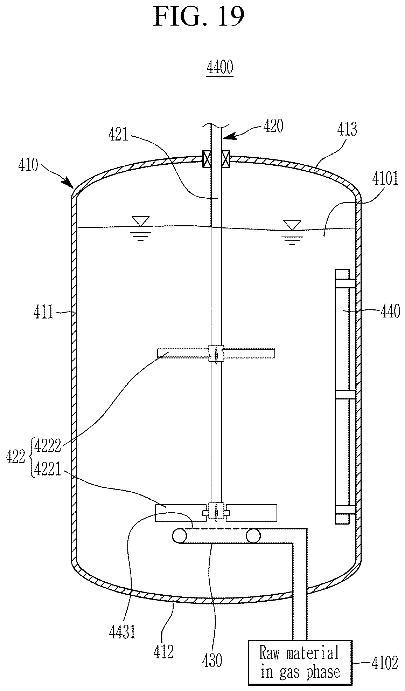

FIG. 19 is a cross-sectional view of a batch reactor according to Comparative Example 4. With reference to FIG. 19, in a batch reactor 4400 of Comparative Example 4, a sparger 4430 is formed as a ring type with spray ports 4431 and is disposed the lower side of the impeller 422 to supply the raw material 4102 in the gas phase to the amine salts 4101.

The impeller 422 is rotationally driven on the upper side of the raw material 4102 in the gas phase sprayed from the sparger 4430 to flow the amine salts 4101. Therefore, the raw material 4102 in the gas phase sprayed to the spray ports 4431 of the sparger 4430 is mixed in the amine salts 4101 while flowing by the flow of the amine salts 4101. Compared to Embodiments 4-1, 4-2, and 4-3, Comparative Example 4 has degraded distribution performance of the raw material 4102 in the gas phase to the amine salts 4101.

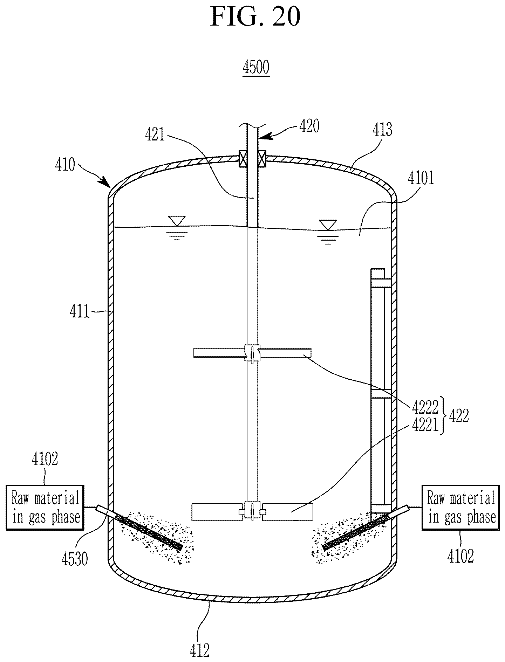

FIG. 20 is a cross-sectional view of a batch reactor according to Embodiments 4-4 of the present disclosure. With reference to FIG. 20, in a batch reactor 4500 of Embodiment 4-4, two spargers 4530 are provided and installed in the sidewall portion 411 of the reactor 410 at the lower end of the reactor 410.

The sparger 4530 is formed as a sintered porous type. The sintered porous type sparger 4530 forms pores over the entire length within the reactor 410 to allow for uniform distribution of the raw material 4102 in the gas phase in the circumferential direction.

In the stirrer of the present disclosure, the impeller may be installed with a radial-type impeller or an axial-type impeller at the lower end and one, two or more, or a plurality of impellers of the same or different types may be provided at the top of the impeller installed at the lower end. In addition, in the stirrer, the impellers may be provided alternately up to the n^{th} stage, which requires a radial-type impeller and an axial-type impeller. The various drawings corresponding to these cases are omitted.

### Table

Table 4 shows the gas holdup for Embodiment 4-1, Embodiment 4-2, Embodiment 4-3, Embodiment 4-4, and Comparative Example 4.

**(Table 4)**

| | Embodiment 4-1 | Embodiment 4-2 | Embodiment 4-3 | Embodiment 4-4 | Comparative Example4 |
|---|---|---|---|---|---|
| Type | Axially spaced pipes | Guide path in rotational shaft of stirrer | Circumferentially spaced pipes | Sintered porous type | Rings |
| Number | Plural | Plural | Plural | Plural | - |
| Gas holdup (%) | >10 | >15 | >10 | >15 | >5 |
| Reaction time (hr) | 13.9 | 9.5 | 11.2 | 10.6 | 14.3 |

### Embodiment 4-1

The amine salts 4101 were contained in the batch reactor 4100 of FIG. 16 , and phosgene, which is the raw material 4102 in the gas phase, was supplied to the amine salts 4101 by the sparger 430 to produce xylylene diisocyanate. In Embodiment 4-1, two stages of the impeller 432 are used, and two stages of the pipe type spargers 430 are used along the axial direction. The stirrer 420 was rotated at 50 to 500 rpm.

### Embodiment 4-2

The amine salts 4101 were contained in the batch reactor 4200 of FIG. 17 , and phosgene, which is the raw material 4102 in the gas phase, was supplied to the amine salts 4101 by the sparger 4230 to produce xylylene diisocyanate. In Embodiment 4-2, two stages of impeller 422 are used, and two stages of bubble formation portion 435 are provided in the guide path 434 formed on the rotational shaft 421. The stirrer 420 was rotated at 50 to 500 rpm.

### Embodiment 4-3

The amine salts 4101 were contained in the batch reactor 4300 of FIG. 18 , and phosgene, which is the raw material 4102 in the gas phase, was supplied to the amine salts 4101 by the sparger 4330 to produce xylylene diisocyanate. In Embodiment 4-3, two stages of impeller 422 are used, and two pipe type spargers 4330 are used along the circumferential direction. The stirrer 420 was rotated at 50 to 500 rpm.

### Embodiment 4-4

The amine salts 4101 were contained in the batch reactor 4500 of FIG. 20 , and phosgene, which is the raw material 4102 in the gas phase, was supplied to the amine salts 4101 by the sparger 4530 to produce xylylene diisocyanate. In Embodiment 4-4, two stages of impeller 422 are used, and two sintered porous type spargers 4530 are used along the circumferential direction. The stirrer 420 was rotated at 50 to 500 rpm.

### Comparative Example 4

The amine salts 4101 were contained in the batch reactor 4400 of FIG. 19 , and phosgene, which is the raw material 4102 in the gas phase, was supplied to the amine salts 4101 by the sparger 4430 to produce xylylene diisocyanate. In Comparative Example 4, two stages of impeller 432 are used, and one ring type sparger 4430 is used at the lower side of impeller 422. The stirrer 420 was rotated at 50 to 500 rpm.

### Experimental Example 4

The gas holdups of Embodiment 4-1, Embodiment 4-2, Embodiment 4-3, Embodiment 4-4, and Comparative Example 4 were measured. In Embodiment 4-1, the gas holdup exceeds 10 % for a reaction time of 13.9 hours, in Embodiment 4-2, the gas holdup exceeds 15 % for a reaction time of 9.5 hours, in Embodiment 4-3, the gas holdup exceeds 10 % for a reaction time of 11.2 hours, and in Embodiment 4-4, the gas holdup exceeds 15 % for a reaction time of 10.6 hours, whereas in Comparative Example 4, the gas holdup exceeds 5 % for a reaction time of 14.3 hours.

That is, Embodiments 4-1, 4-2, 4-3, and 4-4 exhibit an increased gas holdup despite a shorter reaction time compared to Comparative Example 4. Therefore, compared to Comparative Example 4, Embodiments 4-1, 4-2, 4-3, and 4-4 may increase the amount of phosgene, which is the raw material 4102 participating in the reaction, to improve reactivity of the aliphatic isocyanate using phosgene.

While the exemplary embodiments of the present disclosure have been described above, the present disclosure is not limited thereto, and various modifications can be made and carried out within the scope of the claims, the detailed description of the disclosure, and the accompanying drawings, and also fall within the scope of the disclosure.

## Claims

1. A batch reactor comprising: a reactor receiving a solvent in a liquid phase;
a sparger configured to supply a raw material in a gas phase into the reactor; and
a stirrer having an impeller on a rotational shaft installed in the reactor in a height direction to stir the solvent and the raw material,
wherein the impeller comprises
a radial-type impeller and an axial-type impeller.

2. The batch reactor of claim 1, wherein
the radial-type impeller is provided
in one stage at a lower end of the rotational shaft,
and wherein the axial-type impeller is provided
in one or more stages on the rotational shaft at an upper side of the radial-type impeller.

3. The batch reactor of claim 1, wherein
the axial-type impeller is provided
in one stage at a lower end of the rotational shaft,
and wherein the radial-type impeller is provided
in one or more stages on the rotational axis at an upper side of the axial-type impeller.

4. The batch reactor of claim 1, wherein
the reactor comprises
a baffle disposed in parallel with the rotational axis and installed on an inner surface of the reactor.

5. The batch reactor of claim 4, wherein
the baffle has
a plate shape or a rod shape.

6. The batch reactor of claim 1, wherein
the raw material is hydrogen chloride.

7. The batch reactor of claim 1, wherein
hydrogen chloride, which is the raw material, is supplied to produce xylylene diisocyanate.

8. A batch reactor comprising: a reactor receiving a solvent in a liquid phase;
a stirrer having an impeller on a rotational shaft installed in the reactor in a height direction and rotating; and
a sparger allowing a raw material to be distributed to the solvent in the reactor by an operation of the stirrer so that hydrogen chloride, which is the raw material in a gas phase, is supplied to the solvent to produce xylene diisocyanate,
wherein the sparger is configured to
supply the raw material in the gas phase in at least one direction of an axial direction of the stirrer and a circumferential direction of the reactor.

9. The batch reactor of claim 8, wherein
the sparger
is provided in plural along a circumferential direction of the stirrer and installed on a sidewall portion of the reactor at a predetermined interval.

10. The batch reactor of claim 8, wherein
the sparger comprises:
a pipe installed in the reactor in a direction in which a length direction of the sparger intersects an axial direction of the stirrer; and
a bubble formation portion provided at an end of the pipe to supply the raw material in the gas phase in a bubble state toward the impeller.

11. The batch reactor of claim 8, wherein
the sparger
is provided as one sparger at a lower end of the reactor and installed on a sidewall portion of the reactor.

12. The batch reactor of claim 11, wherein
the sparger comprises:
a pipe installed in the reactor with a length direction thereof being oriented in a direction of a diameter of the reactor; and
a bubble formation portion provided at an end of the pipe to supply the raw material in the gas phase in a bubble state toward a downstream side of the impeller.

13. The batch reactor of claim 8, wherein
the sparger comprises:
a ring provided in a circular shape on the lower side of the stirrer; and
a bubble formation portion provided on an upper side of the ring and configured to supply the raw material in the gas phase in a bubble state toward a downstream side of the impeller.

14. The batch reactor of claim 8, wherein
the sparger
is formed as a sintered porous type to supply the raw material in the gas phase in a bubble state towards the impeller.

15. The batch reactor of claim 8, wherein
the impeller comprises:
a radial-type impeller or an axial-type impeller installed at a lower end; and
one or a plurality of impellers of the same or different type installed on a top of the impeller installed on the lower end.

16. A batch reactor comprising: a reactor receiving amine salts in a slurry phase;
a sparger configured to supply phosgene, which is a raw material in a gas phase, to the reactor; and
a stirrer having an impeller on a rotational shaft installed in the reactor in a height direction to stir the amine salts and the phosgene to produce xylylene diisocyanate,
wherein the impeller
is disposed in a plurality of stages on the rotational shaft.

17. The batch reactor of claim 16, wherein
the impeller
is formed as at least one of a radial-type impeller and an axial-type impeller.

18. The batch reactor of claim 16, wherein
the impeller comprises
a radial-type impeller provided in one stage at a lower end of the rotational shaft; and
an axial-type impeller provided in at least two stages on the rotational shaft on an upper side of the radial-type impeller.

19. The batch reactor of claim 16, wherein
the impeller comprises
a radial-type impeller provided in one stage at a lower end of the rotational shaft; and
an axial-type impeller provided in one stage on the rotational shaft on an upper side of the radial-type impeller.

20. The batch reactor of claim 16, wherein
the sparger
is installed to supply the raw material in the gas phase in an axial direction of the stirrer and in a circumferential direction of the reactor.

21. The batch reactor of claim 20, wherein
the sparger comprises:
a pipe installed on a sidewall portion of the reactor in a direction in which a length direction of the sparger intersects an axial direction of the stirrer; and
a bubble formation portion provided with a plurality of spray ports at an end of the pipe to supply the raw material in the gas phase in a bubble state toward the impeller.

22. A batch reactor comprising: a reactor receiving amine salts in a slurry phase;
a stirrer having an impeller on a rotational shaft installed in the reactor in a height direction and rotating; and
a sparger configured to supply a raw material in a gas phase to the amine salts in the reactor to allow the raw material to be distributed to the amine salts by an operation of the stirrer,
wherein the sparger
is provided in plural along one direction of an axial direction of the stirrer and a circumferential direction of the reactor to supply the raw material in the gas phase.

23. The batch reactor of claim 22, wherein
the sparger
is installed on a sidewall portion of the reactor at a predetermined interval along the axial direction of the stirrer.

24. The batch reactor of claim 22, wherein
the sparger comprises:
a pipe installed in the reactor in a direction in which a length direction thereof intersects an axial direction of the stirrer; and
a bubble formation portion provided at an end of the pipe to supply the raw material in the gas phase in a bubble state toward the impeller.

25. The batch reactor of claim 22, wherein
the sparger comprises:
a guide path penetratively formed within the rotational shaft; and
a bubble formation portion penetrating the rotational shaft and connected to the guide path to supply the raw material in the gas phase in a bubble state.

26. The batch reactor of claim 25, wherein
the impeller
is provided in a plurality of stages on the rotational shaft,
and wherein the bubble formation portion
is provided on a lower side of each of the impellers.

27. The batch reactor of claim 22, wherein
the sparger
is installed on a sidewall portion of the reactor at a predetermined interval along the circumferential direction of the reactor at a lower end of the reactor.

28. The batch reactor of claim 27, wherein
the sparger includes
a pipe installed in the reactor with a length direction thereof being oriented in a direction of a diameter of the reactor; and
a bubble formation portion provided at an end of the pipe to supply the raw material in the gas phase in a bubble state toward a downstream side of the impeller.

29. The batch reactor of claim 22, wherein
phosgene, which is the raw material, is supplied to produce xylylene diisocyanate.

30. The batch reactor of claim 27, wherein
the sparger
is formed as a sintered porous type and installed in the reactor in a direction in which a length direction thereof intersects an axial direction of the stirrer to supply the raw material in the gas phase towards the impeller in a bubbled state.
